Europäisches Patentamt

European Patent Office

Office européen des brevets

(19)

(11) Numéro de publication : **0 389 381 B1**

# (12) FASCICULE DE BREVET EUROPEEN

(45) Date de publication du fascicule du brevet :
**02.11.94 Bulletin 94/44**

(51) Int. Cl.⁵ : **G01N 33/577,** G01N 33/543,
C12Q 1/00, C12Q 1/28

(21) Numéro de dépôt : **90400805.9**

(22) Date de dépôt : **23.03.90**

(54) **Trousse et méthode de dosage enzymatique applicables à des cellules entières.**

(30) Priorité : **24.03.89 FR 8903943**

(43) Date de publication de la demande :
**26.09.90 Bulletin 90/39**

(45) Mention de la délivrance du brevet :
**02.11.94 Bulletin 94/44**

(84) Etats contractants désignés :
**AT BE CH DE DK ES FR GB GR IT LI LU NL SE**

(56) Documents cités :
**EP-A- 0 054 676
EP-A- 0 179 007
EP-A- 0 183 048
EP-A- 0 224 134
WO-A-84/03151**

(56) Documents cités :
**WO-A-88/06918
WO-A-88/08538
US-A- 4 770 995
CHEMICAL ABSTRACTS, vol. 103, no. 21, 25
novembre 1985, Columbus, OH (US); U. LIPPI
et al., p. 317, no. 174392q**

(73) Titulaire : **ELF SANOFI
32-34, rue Marbeuf
F-75008 Paris (FR)**

(72) Inventeur : **Carriere, Dominique
29 Lotissement La Colline
F-34160 Castries (FR)**

(74) Mandataire : **Gillard, Marie-Louise et al
Cabinet Beau de Loménie
158, rue de l'Université
F-75340 Paris Cédex 07 (FR)**

EP 0 389 381 B1

Il est rappelé que : Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

## Description

La présente invention concerne une trousse et une méthode de dosage enzymatique utilisant cette trousse, pour le dosage d'enzymes endogènes caractéristiques de populations ou de sous-populations de cellules. La méthode de dosage enzymatique et la trousse correspondante sont également destinées au dosage des cellules elles-mêmes par l'intermédiaire du dosage de leurs enzymes endogènes. Ces dosages sont applicables au diagnostic.

Le procédé selon l'invention consiste à immobiliser de façon spécifique par immunocapture sur un support solide une population cellulaire puis à doser une enzyme endogène spécifique de cette population ou d'une sous-population cellulaire en mettant en oeuvre un substrat approprié de cette enzyme et éventuellement les cofacteurs ou les substances auxiliaires nécessaires.

Selon la présente invention, dans la description et les revendications qui vont suivre, on appelle enzyme endogène, une enzyme contenue dans le cytoplasme de la cellule et dont le substrat est une molécule de bas poids moléculaire capable de pénétrer à l'intérieur de la cellule pour y être transformée par l'enzyme, en libérant dans le milieu un produit de réaction coloré ou fluorescent qui permet la mesure finale d'un signal proportionnel à l'activité de l'enzyme.

L'immunocapture des cellules est un procédé qui consiste à retenir les cellules sur un support solide par liaison entre un ou plusieurs antigènes de surface de ces cellules et un ou plusieurs anticorps monoclonaux spécifiques de ce ou de ces antigènes.

La connaissance des antigènes ou marqueurs de la surface cellulaire a fait d'énormes progrès avec la mise au point de l'hybridation lymphocytaire et la découverte des anticorps monoclonaux par KOEHLER et MILSTEIN, (Nature, 1975, 256 , 495-497). Les anticorps monoclonaux ont notamment permis la mise en évidence et l'analyse de marqueurs de surface ou antigènes membranaires de cellules d'origines les plus variées. Ces marqueurs (ou antigènes) peuvent être de différentes natures : protéines, glycoprotéines ou glycolipides. Les caractérisations recherchées s'adressent alors principalement à des marqueurs de tissu ou d'organe, à des marqueurs d'états de différenciation ou d'activation de cellules normales et à l'identification ou au typage de cellules normales ou cancéreuses. Un domaine d'application particulièrement important est l'étude des lignées cellulaires de l'hématopoïèse (érythrocytaire, megakaryocytaire, granulocytaire, monocytaire, lymphocytaire).

Ainsi, par exemple, les anticorps monoclonaux ont permis de préciser les caractéristiques de surface respectives des lymphocytes T et B. Les marqueurs correspondants seuls ou en combinaison identifient des stades de différenciation et de spécialisation fonctionnelle des lymphocytes. Par convention internationale, les marqueurs de surface des leucocytes humains ont été classés dans des groupes ou classes de différenciation (CD) définis lors du Sous-Comité IUIS-OMS, 1984 et décrits dans Bulletin de l'Organisation Mondiale de la Santé, 1984, 62 (5), 813-815.

Les anticorps monoclonaux sont aujourd'hui des outils irremplaçables de la biologie clinique appliquée aux analyses cellulaires.

Il existe des méthodes de dénombrement de cellules qui utilisent le marquage de leurs antigènes de surface mais ces méthodes sont souvent longues, laborieuses, difficiles à mettre en oeuvre, et les résultats en sont parfois aléatoires.

Un groupe de méthodes de mesure des antigènes est basé sur l'évaluation quantitative des marqueurs de surface de la population cellulaire globale. Ces méthodes permettent la mesure des antigènes par un marquage soit direct, soit indirect qui est alors réalisé le plus souvent en deux, trois ou quatre étapes. Dans tous les cas le réactif employé lors de la dernière étape du marquage porte une sonde qui est soit de nature isotopique, Iode 125 par exemple, pour un dosage de type immunoradiométrique (BROW et Coll. J. Immunol. Methods, 1979, 31, 201 - STOCKER et HEUSSER J. Immunol. Methods, 1979, 26, 87-95) soit une enzyme pour un dosage de type immunoenzymométrique, le plus souvent la peroxydase, la phosphatase alcaline ou la bêta-galactosidase, (VAN LEUVEN et Coll., J. Immunol. Methods, 1978, 23, 109-116 - MORRIS Transplantation, 1983, 36(6), 719 - BAUMGARTEN J. Immunol. Methods, 1986, 94, 91-98).

On notera que les enzymes utilisées dans de telles méthodes sont des réactifs analytiques apportés au cours du dosage sous forme conjuguée à un anticorps qui reconnait un antigène exposé à la face externe de la membrane cellulaire. Ces enzymes ne peuvent donc se confondre avec les enzymes endogènes de la cellule qui font partie de son équipement enzymatique naturel.

Ces méthodes sont assez incommodes, laborieuses et hasardeuses à appliquer en raison de la nécessité de nombreux lavages et centrifugations du matériel cellulaire ; il est nécessaire parfois de prélever le milieu coloré résultant de la réaction enzymatique en vue de la mesure spectrophotométrique finale ; enfin la fixation chimique des cellules, qui est le plus souvent utilisée, est à l'origine de la destruction irréversible de certains antigènes particulièrement sensibles aux fixateurs chimiques usuels tels que glutaraldéhyde ou méthanol (DROVER et Coll. J. Immunol. Methods, 1986, 90, 275-281).

L'immunocapture de cellules sur un support solide est décrite dans la demande de brevet WO 86/02091 dans laquelle le but est d'éliminer des cellules indésirables dans des échantillons de moelle osseuse destinée à des transplantations. Dans cette demande de brevet, la capture des cellules est réalisée sur des microbilles flottantes et nécessite que l'anticorps utilisé soit relié au support solide par une structure macromoléculaire complexe, appelée réseau-relais, capable d'assurer une orientation privilégiée de l'anticorps par rapport à celle de l'antigène cellulaire correspondant. Aucune application de la technique au dosage quantitatif d'une enzyme cellulaire n'est indiquée dans cette demande.

L'immunocapture de cellules est également décrite dans la demande de brevet WO 84/03151 pour une application analytique. Dans cette demande, le but est d'identifier les groupes tissulaires auxquels appartiennent les cellules examinées (opération généralement dénommée typage HLA). La capture des cellules est effectuée grâce à des anticorps disposés selon une géométrie particulière sur des supports très spécialisés (lamelles de microscope). Les résultats sont obtenus par simple observation visuelle du support et conduisent à des réponses en "tout ou rien".

Ainsi, les systèmes d'immunocapture de cellules décrits jusqu'à ce jour ne conduisent pas à des applications analytiques permettant la détermination quantitative d'un marqueur spécifique de la cellule et en particulier une enzyme constitutive de la cellule.

La méthode de dosage qui fait l'objet de la présente invention, possède des avantages considérables par rapport à toutes les techniques antérieurement connues et utilisées car elle permet de mesurer de façon quantitative toute enzyme endogène d'une population cellulaire. Ce dosage est réalisé sur des cellules qui n'ont subi au moment de leur capture spécifique aucune intervention chimique ou physique et qui sont donc dans leur état d'intégrité physiologique. De plus, la méthode de dosage selon l'invention possède les caractéristiques de très haute spécificité, propres aux systèmes à double reconnaissance mettant en oeuvre deux marqueurs différents spécifiques portés par la même cellule, l'un étant un antigène choisi pour immunocapturer les cellules que l'on veut analyser, l'autre étant une enzyme endogène présente dans les cellules qui ont été capturées. Cette méthode est simple, rapide et reproductible. Elle est tout à fait adaptée à l'analyse d'un grand nombre d'échantillons ce qui permet son utilisation à des fins de diagnostic dans les laboratoires de biologie clinique à grand débit.

Ainsi la présente invention se rapporte à une trousse pour le dosage d'au moins une enzyme endogène caractéristique d'une population ou sous-population cellulaire, comprenant comme composants :

a) un support solide sur lequel sont fixés par liaison covalente ou adsorption physique un ou plusieurs anticorps monoclonaux dirigés contre des antigènes de surface de la population cellulaire examinée et destinés à immobiliser sur le support les cellules parmi lesquelles se trouvent celles de la sous-population qui possède l'enzyme à doser ;

b) une solution contenant le substrat de l'enzyme pour révéler l'activité de l'enzyme, ledit substrat étant une molécule de bas poids moléculaire capable de pénétrer à l'intérieur de ladite population cellulaire.

c) une solution tampon destinée au lavage du support solide.

La trousse peut en outre contenir une solution d'un réactif chromogène pour révéler l'activité de l'enzyme, et des échantillons permettant l'étalonnage et le contrôle de qualité du dosage.

Le terme cellule, utilisé dans la présente description et dans les revendications qui vont suivre, inclut les cellules humaines ou animales et notamment les cellules sanguines y compris les plaquettes.

La méthode de dosage selon l'invention s'applique à des cellules entières, c'est à dire non lysées.

Ces cellules n'ont subi au moment de leur immunocapture aucune intervention physique ou chimique et elles sont mises en oeuvre dans un état d'intégrité physiologique complète. Cette situation constitue la meilleure garantie d'intégrité de l'antigène utilisé pour la capture et de l'enzyme endogène choisie comme cible de dosage.

Comme support solide, on peut utiliser tout dispositif adapté à la manipulation de suspensions cellulaires et préférentiellement des tubes, des supports magnétiques particulaires ou des plaques rigides ou souples de microtitration en polyéthylène, polystyrène, chlorure de polyvinyle ou nitrocellulose comportant des micropuits. Les anticorps monoclonaux destinés à l'immobilisation des cellules peuvent être fixés sur les supports solides soit par liaison chimique covalente, soit par adsorption physique selon les techniques classiques bien connues de l'homme de l'art, telles que celles décrites par STOCKER et HEUSSER (J. Immunol. Methods, 1979, vol. 26, p. 87-95). Avantageusement, le support peut être au préalable saturé à l'aide d'une protéine.

Selon l'invention, le ou les anticorps monoclonaux fixés sur le support solide doivent permettre l'immunocapture de la population cellulaire parmi laquelle se trouve la ou les populations cellulaires qui contiennent l'enzyme à doser. Lorsque cette population est constituée de cellules humaines, les anticorps monoclonaux préférés pour l'immunocapture sont les anticorps anti-HLA de classe I qui sont spécifiques de la partie commune des antigènes HLA A, B et C présents sur les leucocytes et de nombreuses autres lignées cellulaires de l'organisme. De même, les anticorps monoclonaux spécifiques des antigènes des classes de différenciation qui ont été déterminés sur les leucocytes sont préférés. Parmi ces anticorps, celui dénommé S-classe I, commercialisé par BYOSIS, est particulièrement préféré.

Dans d'autres cas où les cellules examinées sont des cellules humaines et dans tous les cas lorsque ces cellules ne sont pas humaines, des anticorps monoclonaux appropriés au type de cellules examinées peuvent également être utilisés pour l'immunocapture selon l'invention.

Le substrat de l'enzyme endogène à doser et les réactifs sont choisis de sorte que le produit final de la réaction ou de la séquence de réactions provoquée par l'enzyme et mettant en oeuvre ces substances soit :
- ou bien une substance colorée ou fluorescente qui diffuse dans le milieu liquide environnant les cellules et qui fait l'objet de la mesure finale spectrophotométrique ou fluorimétrique, respectivement,
- ou bien une substance colorée insoluble qui se dépose sur les cellules et les parois sur lesquelles elles sont fixées et qui peut faire l'objet, soit d'une mesure photométrique par réflexion, soit d'une évaluation à l'oeil, éventuellement par rapport à une gamme de teintes étalonnées.

Ainsi l'utilisation d'un chromogène n'est pas nécessaire lorsque le substrat seul permet d'obtenir une substance colorée ou fluorescente.

La trousse de dosage contient comme composant additionnel une solution tampon destinée au lavage du support solide après immobilisation des cellules.

La trousse de dosage peut aussi contenir, comme autres composants additionnels, les échantillons nécessaires à l'étalonnage du dosage ainsi qu'au contrôle de qualité du dosage.

La présente invention a également pour objet un procédé pour le dosage des enzymes endogènes d'une population ou d'une sous-population cellulaire caractérisé en ce qu'il consiste :
- à immobiliser la population cellulaire comprenant la sous-population contenant l'enzyme endogène sur un support solide en utilisant un ou plusieurs anticorps monoclonaux, préalablement fixé par liaison covalente ou par adsorption physique sur ledit support et capable de reconnaître un antigène présent à la surface des cellules ;
- à observer une période d'incubation pour permettre l'immunocapture ;
- à laver le support solide pour éliminer les cellules non immobilisées ;
- à ajouter le ou les réactifs nécessaires(substrat et le cas échéant chromogène) pour révéler l'activité de l'enzyme endogène ;
- à lire les résultats par mesure des signaux lumineux (coloration ou fluorescence) en se référant éventuellement à une gamme d'étalonnage.

Ainsi la révélation de l'enzyme endogène à doser, appartenant à la population cellulaire immobilisée, ou à une de ses sous-populations est effectuée directement par un substrat spécifique de cette enzyme endogène, puis le dosage proprement dit de l'enzyme endogène est réalisé par mesure photométrique en transmission ou en réflexion, ou par mesure de l'émission de fluorescence.

La trousse et le procédé de dosage selon l'invention sont particulièrement simples car, lorsqu'on dispose de supports d'immunocapture préparés à l'avance, ils ne font intervenir que la ou les solutions contenant les réactifs spécifiques nécessaires à la mesure de l'activité de l'enzyme (substrat et éventuellement chromogène).

La trousse de dosage et le procédé enzymatique selon l'invention s'appliquent de façon préférentielle au dosage des enzymes endogènes des éléments figurés du sang humain notamment les leucocytes, plus particulièrement les granulocytes.

La trousse de dosage et le procédé enzymatique selon l'invention permettent de mesurer des signaux (lumière absorbée ou émise) dépendant à la fois du nombre de cellules présentes dans la population cellulaire examinée et de la concentration de l'enzyme endogène mesurée dans ces cellules. La mesure de ces signaux permet une évaluation quantitative de l'activité des molécules de cette enzyme qui sont contenues dans les cellules de la population ou sous-population cellulaire examinée.

Une application de l'invention apparait en choisissant comme support solide une plaque de microtitration. La trousse de dosage et le procédé enzymatique selon l'invention peuvent alors avantageusement être utilisés pour le dosage sur une seule plaque d'une série d'enzymes endogènes caractéristiques de diverses sous-populations constitutives de la population cellulaire examinée. Pour cette application, on peut utiliser d'une part, des plaques de microtitration prêtes à l'emploi sur lesquelles ont été fixés à l'avance un ou plusieurs anticorps monoclonaux capables de retenir toutes les cellules de la population examinée et disposer, d'autre part, d'une série de substrats spécifiques chacun d'une enzyme endogène caractéristique de l'une des sous-populations à évaluer. Ainsi, sur un même support, on peut obtenir le dosage quantitatif de toutes les enzymes endogènes nécessaires à la caractérisation des sous-populations choisies.

A titre d'application de l'invention, on peut citer le cas des leucocytes humains, pour lesquels il existe notamment la sous-population des cellules dites polynucléaires ou granulocytes.

4

La présence de granulocytes humains peut être évaluée dans le sang ou dans les urines. Ainsi, dans le cas d'infections urinaires telles que cystites ou pyélonéphrites, il est particulièrement intéressant de disposer d'une méthode simple pour le dosage des granulocytes dans les urines.

La trousse de dosage et le procédé enzymatique selon l'invention peuvent être utilisés pour le dosage des granulocytes. Dans ce cas, on effectue l'immobilisation spécifique des granulocytes de l'échantillon examiné sur un support solide, et on effectue le dosage d'une enzyme endogène caractéristique des granulocytes en utilisant un substrat approprié. La myéloperoxydase est une enzyme endogène des granulocytes et son dosage est effectué en utilisant l'eau oxygénée comme substrat de la péroxydase.

Pour obtenir l'immobilisation spécifique des granulocytes on utilise les anticorps monoclonaux anti-CD15 fixés sur un support solide, tel que les parois des micropuits des plaques de microtitration, par exemple.

Les plaques ainsi préparées peuvent être lyophilisées et stockées de préférence à 4°C. Cette étape peut être réalisée industriellement et l'on pourra ainsi disposer de plaques prêtes a l'emploi pour les trousses de dosage applicables aux granulocytes de l'échantillon de sang ou d'urine examiné.

Les échantillons contenant les cellules à doser et provenant du sang ou de tout liquide biologique approprié, normal ou pathologique, notamment l'urine, peuvent être utilisés tels quels ou après préparation, notamment après concentration.

Des parties aliquotes de la suspension cellulaire appropriée sont placées au contact du support solide, par exemple dans des tubes contenant le support microparticulaire d'immunocapture ou dans les micropuits d'une plaque de microtitration préalablement préparée. Après lavage, on ajoute la solution faisant partie de la trousse de dosage et contenant le substrat spécifique de l'enzyme endogène caractéristique de la population cellulaire visée.

La durée nécessaire pour l'immobilisation des cellules est de préférence inférieure ou égale à 15 minutes. Pendant ce délai, le support solide peut être centrifugé pour améliorer l'immobilisation des cellules. Le support solide, le tube ou la plaque de microtitration par exemple, est ensuite lavé pour éliminer les cellules non fixées.

L'apparition d'un produit coloré ou fluorescent est obtenue en ajoutant au support solide sur lequel a été fixée la population cellulaire portant l'enzyme endogène à doser une solution contenant le substrat de l'enzyme et, si nécessaire, un ou plusieurs réactifs auxiliaires permettant d'obtenir finalement comme produit de réaction, soit un produit coloré soluble dans le milieu, soit un produit coloré insoluble, soit un produit fluorescent soluble, comme cela a été expliqué précédemment. On mesure ensuite le signal lumineux provenant des échantillons ainsi traités, à l'aide de l'appareillage adapté à chaque cas : photomètre en transmission ou en réflexion ou fluorimètre respectivement. Lorsque le support solide est une plaque de microtitration, la lecture du signal lumineux peut être effectuée séquentiellement dans tous les puits d'une même plaque par l'emploi de lecteurs automatisés d'usage courant dans les laboratoires de biologie, tels que par exemple le lecteur de plaque Titertek ou le lecteur de plaque Fluoroscan, pour les lectures spectrophotométriques ou fluorométriques respectivement.

Les réactifs utilisés pour révéler la peroxydase endogène ou la myéloperoxydase endogène contiennent de l'eau oxygénée, substrat de l'enzyme, et un chromogène approprié, par exemple de l'orthophénylènediamine ou l'acide azino-2,2′ bis (éthyl-3 benzothiazoline sulfonique-6) ou ABTS pour obtenir un produit final de réaction coloré et soluble dans le milieu ou bien la diamino-3,3′ benzidine ou l'amino-3 éthyl-9 carbazole ou le chloro-4 alpha-naphtol pour obtenir un produit final de réaction insoluble, ou bien l'acide parahydroxyphényl propionique pour obtenir un produit de réaction fluorescent soluble dans le milieu.

Selon une forme préférentielle, la trousse selon l'invention, pour le dosage de myéloperoxydase caractéristique des granulocytes comprend :

a) une plaque de microtitration dans les puits de laquelle ont été fixés un ou plusieurs anticorps monoclonaux anti-granulocytes ;

b1) une solution contenant de l'eau oxygénée, substrat de l'enzyme, dans un tampon approprié.

b2) une solution contenant le chromogène utilisé pour révéler l'expression de l'activité de l'enzyme et éventuellement un réactif qui améliore la perméabilité de la membrane cellulaire.

Selon une autre forme préférentielle de réalisation, la trousse selon l'invention pour le dosage de myéloperoxydase caractéristique des granulocytes comprend comme support solide des tubes ou encore des supports magnétiques particulaires sur lesquels ont été fixés un ou plusieurs anticorps monoclonaux antigranulocytes.

Les résultats du dosage des enzymes endogènes selon l'invention peuvent être exprimés selon toute modalité appropriée à l'examen effectué. Plus particulièrement on peut exprimer ces résultats en activité totale d'une enzyme endogène particulière présente dans un volume donné de l'échantillon examiné (par exemple par microlitre de sang).

Pour déterminer l'activité d'une enzyme endogène particulière, dans l'échantillon examiné, on utilisera de préférence une gamme d'étalonnage constituée de cellules ou de préparations appropriées, contenant l'enzyme endogène à doser et qui auront été préalablement calibrées par une méthode de référence connue. Ces étalons seront de préférence constitués soit par des cellules identiques dans leur provenance aux cellules qui feront l'objet du dosage, soit par des cellules de lignées cellulaires établies contenant l'enzyme endogène recherchée, soit par des préparations acellulaires contenant l'enzyme choisie.

Ces étalons sont alors traités exactement comme les échantillons à examiner. Les signaux qui en découlent servent à construire une gamme d'étalonnage à laquelle sont rapportés les signaux mesurés avec les échantillons à examiner. Les calculs ultérieurs sont classiques.

La méthode de dosage enzymatique selon l'invention est simple, rapide et reproductible. Son utilisation est tout à fait adaptée à l'analyse d'un grand nombre d'échantillons. Pour en comprendre les avantages par comparaison avec les autres méthodes décrites, il convient d'en analyser les différentes étapes.

L'immobilisation des cellules sur le support solide est la phase du dosage qui présente habituellement le plus de difficultés ou qui est la plus critique dans sa réalisation. Le moyen souvent utilisé est la fixation chimique des cellules par le glutaraldéhyde ou le méthanol dans des cupules traitées ou non à la poly-L-lysine (VAN LEUVEN F. et Coll., J. Immunol. Methods, 1978, 23, 109). Toutefois, les fixations chimiques ainsi mises en oeuvre peuvent diminuer ou même supprimer la détection spécifique recherchée ou au contraire induire des marquages faussement positifs de cellules ce qui est un inconvénient très grave (DROVER et MARSHALL J. Immunol. Methods, 1986, 90, 275-281).

De plus la réalisation du procédé par fixation chimique nécessite plusieurs étapes : la centrifugation des cellules, la préparation du mélange de fixateur, la fixation puis les lavages des cellules fixées.

La dessiccation des cellules à 37°C, suivie ou non d'une fixation au méthanol dans les micropuits a aussi été proposée (BAUMGARTEN, J. Immunol. Methods, 1986, 94, 91-98). En fait, la déshydratation des cellules à 37°C, peut altérer certains antigènes fragiles qui pourraient être utiles à l'immunocapture des cellules ainsi que les enzymes endogènes à doser.

De plus, la reproductibilité de ce procédé est incertaine ; en effet, la décantation des cellules dans les micropuits du dosage et la dessiccation des cellules peuvent varier d'une expérience à l'autre. Enfin, ce dosage est long à réaliser car l'étape de dessiccation des cellules nécessite à elle seule un délai supérieur à 2 heures.

L'immobilisation de populations lymphocytaires a été aussi obtenue grâce à l'emploi d'anticorps polyclonaux adsorbés dans des micropuits (STOCKER et HEUSSER J. Immunol. Methods, 1979, 26, 87-95). Ce procédé permet l'immobilisation de cellules étrangères à la seule population que l'on souhaite analyser, ce qui présente aussi un inconvénient certain.

L'utilisation d'anticorps monoclonaux hautement spécifiques et affins adsorbés ou fixés sur le support solide et notamment dans les puits du dosage, les tubes, ou sur le support particulaire permet la capture exclusive des cellules recherchées, les autres populations cellulaires non retenues étant éliminées au cours des lavages effectués. De plus, aucun agent chimique ou physique ne modifie les caractéristiques des antigènes à cette étape car les différentes opérations destinées à fixer chimiquement ou physiquement les cellules au support sont supprimées.

Ainsi, selon la présente invention, il a été trouvé que l'immobilisation des cellules par des anticorps monoclonaux est un procédé qui permet de simplifier l'étape d'immobilisation des cellules portant l'enzyme à doser, tout en rendant les résultats plus fiables.

Le procédé selon l'invention comportant l'utilisation d'un processus d'immunocapture des cellules entières, sans intervention physique ou chimique sur les cellules, et la mesure dans tout ou partie de ces cellules de l'activité d'une enzyme endogène grâce à l'emploi de son substrat spécifique permet, pour la première fois, le dosage quantitatif sur les cellules elles-mêmes des enzymes endogènes choisies.

Selon l'invention, le marquage direct des cellules immobilisées immunologiquement permet :
- une économie de réactifs ;
- une fiabilité améliorée par diminution du nombre d'étapes et de manipulations;
- un gain de temps;
- la possibilité de traiter en même temps, avec l'emploi exclusif de matériels et d'appareillages usuels, de grands nombres d'échantillons.

La durée nécessaire pour l'immobilisation de la population cellulaire ou de la sous-population cellulaire à doser est courte. Elle est inférieure ou égale à 15 minutes dans le cas du dosage des granulocytes sanguins ou urinaires. De même, la période pour le dosage de l'activité de l'enzyme endogène est inférieure ou égale à 15 minutes.

Après le lavage du support solide, le dosage proprement dit est effectué par l'observation d'un signal précis et simple à mesurer avec des appareillages usuels : absorption ou émission lumineuse.

Ainsi l'ensemble du procédé selon l'invention présente de nombreux avantages : il est rapide, fiable, économique et simple.

On a vérifié que les signaux enregistrés (mesures photométriques) permettent d'obtenir des courbes d'étalonnage régulières et satisfaisantes en fonction du nombre de cellules mises en oeuvre, dans les conditions usuelles de manipulation.

Dans les exemples qui vont suivre, on utilisera indifféremment les termes suivants ou leurs abréviations :
BSA : albumine de sérum bovin, ou sérum albumine bovine
PBS : tampon phosphate salin à pH 7,4

EXEMPLE 1

DOSAGE DE LA MYELOPEROXYDASE DES LEUCOCYTES POLYNUCLEAIRES DU SANG HUMAIN : DOSAGE REALISE EN PLAQUE A MICROTITRATION.

La myéloperoxydase est une enzyme de nature glycoprotéique comprenant une structure hème. Cette enzyme abondante dans les leucocytes polynucléaires du sang humain est impliquée dans les fonctions bactéricides et anti-microbiennes des cellules. (KLEBANOFF S.J., J. Bactériol., 1968, 95, 2131 - DIAMOND et coll., J. Clin. Invest., 1980, 66 908-917).

Le dosage de la myéloperoxydase des polynucléaires selon le procédé de l'invention comprend trois étapes réalisées successivement:

1. la séparation des polynucléaires du sang total,
2. la capture et l'immobilisation sélective des cellules par un anticorps monoclonal préalablement adsorbé dans les micropuits d'une plaque de microtisation,
3. la révélation de l'activité de l'enzyme cellulaire.

a) Préparation de la plaque

On utilise une plaque de microtitration en plastique commercialisée par NUNC (Référence 64394) comportant 96 micropuits. On place par micropuits 200 µl d'une solution contenant l'anticorps monoclonal purifié anti-CD15 (dénommé SMY 15a) utilisé pour immobiliser les leucocytes polynucléaires, c'est à dire pour effectuer leur immunocapture. Cet anticorps commercialisé par BIOSYS, Compiègne, France, sous la référence SMY 15a est utilisé à la concentration de 5 µg/ml dans un tampon phosphate salin à pH 7,4 (PBS).

L'adsorption de l'anticorps monoclonal s'effectue à 4°C pendant 12 heures. On élimine l'anticorps en excès par retournement de la plaque.

On prépare une solution contenant 0,1 % de gélatine et 0,3 % de BSA dans un tampon phosphate salin. On introduit 250 µl de cette solution par micropuits, afin de saturer la surface des puits en protéine, ce qui est obtenu après 1 heure à 37°C ; on lave les plaques 3 fois à l'aide de tampon phosphate salin. Les plaques ainsi préparées sont stockées à 4°C.

b) Séparation des leucocytes polynucléaires.

Le sang est prélevé sur anticoagulant (héparine). Dans un tube à hémolyse de 5 ml, on introduit successivement 2 ml de milieu de séparation MONO-POLY (FLOW réf. 16.980-49) et on dépose 2 ml de sang. Le tube est ensuite centrifugé à 400 x g pendant 40 minutes à température du laboratoire. Il se forme deux anneaux de suspension cellulaire ; l'anneau inférieur contient les polynucléaires qui sont récupérés à l'aide d'une micropipette.

c) Immunocapture cellulaire.

La capture des cellules est réalisée grâce à l'anticorps monoclonal anti-CD15 adsorbé.

On introduit dans les micropuits de la plaque 100 µl de la suspension cellulaire ajustée à 5 x 10⁵ cellules par ml de tampon PBS. Pour améliorer la fixation des cellules sur le support, on centrifuge la plaque pendant 3 minutes à 150 x g après attente de 10 minutes à température ambiante.

d) Révélation et mesure de la myéloperoxydase.

On vide les micropuits par retournement de la plaque. On lave les micropuits 2 fois par 200 µl de tampon PBS ce qui permet d'éliminer les populations cellulaires indésirables telles que les monocytes ou les hématies qui peuvent être à l'origine d'un signal parasite d'absorbance. Après le deuxième lavage, on ajoute dans chaque puits 100 µl du réactif de révélation préparé extemporanément.

Le réactif de révélation est obtenu de la façon suivante : on prépare un tampon citrate 0,1 M en dissolvant de l'acide citrique monohydraté à 2 % dans de l'eau et en ajustant à pH 5 par addition de soude 7N. On réalise ensuite une solution à 2 % de bromure d'héxadécyltriméthyl ammonium (= CETAB, TOUZART et MATIGNON T 5650) dans le tampon. Le réactif perméabilise les membranes cellulaires et améliore l'activité de la myéloperoxydase sur le substrat. A 20 ml de cette solution on ajoute avant emploi 30 mg de dichlorhydrate d'orthophénylènediamine puis 40 µl d'eau oxygénée (substrat de l'enzyme).

Après incubation pendant 10 minutes, on mesure l'absorbance au spectrophotomètre à 450 nm (appareil Titertek Multiskan type 310 C - Flow Laboratories).

Pour une expérience, les absorbances obtenues avec 50 000 cellules sont données dans le tableau 1 ci-dessous :

TABLEAU 1

|  | CELLULES SEULES | CELLULES + réactifs sans CETAB | CELLULES + réactifs avec CETAB |
|---|---|---|---|
| Absorbance | 0,003 | 0,370 | 1,545 |

La présence de CETAB dans le milieu de révélation augmente d'un facteur de 4 la densité optique due à la réaction catalysée par la myéloperoxydase, ce qui permet de réduire le nombre de cellules nécessaires pour le dosage.

EXEMPLE 2.

DOSAGE DE LA MYELOPEROXYDASE DES LEUCOCYTES POLYNUCLEAIRES DU SANG ET DES URINES : DOSAGE REALISE SUR DES BILLES MAGNETIQUES

Cet exemple est destiné à montrer que l'on peut diminuer la durée nécessaire au dosage d'une enzyme endogène, par rapport aux conditions décrites dans l'exemple 1, en modifiant le procédé d'immunocapture des cellules. Le support utilisé pour capturer les cellules est constitué de billes magnétiques DYNABEADS. Les billes (réf. DYN-11001 commercialisées par BYOSIS) portent à leur surface des anticorps anti-immunoglobines de souris. Les billes sont traitées avant usage par une solution d'anticorps monoclonaux anti-CD15 (réf. SMY 15a - BYOSIS France) pendant 12 heures à 4°C. Pour cela, 25 µl de la suspension de billes sont mélangés à 0,5 ml de solution d'anticorps à 100 µg/ml de tampon PBS. Les billes sont ensuite lavées 3 fois avec du tampon PBS puis saturées 12 heures à 4°C par une solution à 0,3 d'albumine sérique bovine (BSA). Les billes prêtes à l'emploi sont conservées à 4°C.

Le dosage de la myéloperoxydase est réalisé dans un tube à hémolyse de 5 ml ; on introduit dans le tube 300 µl de tampon PBS, 25 µl de suspension de billes et 100 µl de sang complet prélevé sur héparinate de lithium (tube VACUTAINER réf. 606 484). Après 5 minutes sous agitation douce, les billles sont séparées grâce à un aimant et les cellules fixées sur les billes sont lavées avec du PBS (5 lavages).

La myéloperoxydase est révélée avec un réactif mixte contenant le substrat (eau oxygénée) et le chromogène (orthophénylènediamine) préparé comme dans l'exemple 1, avec ou sans incorporation de CETAB. L'absorbance est mesurée dans les conditions identiques à celles de l'exemple 1. Les résultats sont donnés tableau 2.

La mesure des myéloperosydases des granulocytes présents dans l'urine au cours des infections urinaires est un élément de diagnostic important des pyélonéphrites et pyuries. On a montré dans la deuxième partie de l'exemple que les particules magnétiques portant le même anticorps monoclonal anti-CD15 sont capables de capturer les leucocytes polynucléaires dans le liquide urinaire. Le dosage est réalisé sur 400 µl du liquide urinaire contenant environ $2 \times 10^5$ granulocytes par ml. Les cellules sont capturées avec 25 µl de suspension de billes, puis traitées dans les mêmes conditions que dans l'exemple précédent. Les résultats sont donnés dans le tableau 2 ci-dessous.

TABLEAU 2

| ABSORBANCES | BILLES + réactif de révélation | BILLES + CELLULES seules | BILLES + CELLULES + réactif de révélation sans CETAB | BILLES + CELLULES + réactif de révélation avec CETAB |
|---|---|---|---|---|
| Sang total | 0,025 | 0,011 | 0,0323 | 1,324 |
| Urine | 0,025 | 0,008 | 0,286 | 1,115 |

Selon le procédé décrit dans cet exemple, le dosage a été effectué directemet sur l'échantillon de sang, puis sur l'échantillon d'urine, sans nécessiter de séparation préalable de la population cellulaire à analyser. Par ailleurs, la période d'incubation est seulement de 5 minutes. Ainsi, en utilisant les billes magnétiques comme support solide, l'ensemble des opérations de dosage est particulièrement simple et rapide.

**Revendications**

1. Trousse pour le dosage d'une enzyme endogène caractéristique d'une population ou d'une sous-population cellulaire comprenant, comme composants :
   a) un support solide sur lequel sont fixés par liaison covalente ou par adsorption physique un ou plusieurs anticorps monoclonaux dirigés contre des antigènes de surface de la population cellulaire à doser;
   b) une solution contenant le substrat de l'enzyme pour révéler l'activité de l'enzyme, ledit substrat étant une molécule de bas poids moléculaire capable de pénétrer à l'intérieur de ladite population cellulaire.
   c) une solution tampon de lavage.

2. Trousse selon la revendication 1 comprenant en outre un réactif chromogène pour révéler l'activité de l'enzyme.

3. Trousse selon l'une des revendications 1 et 2 comprenant en outre des échantillons permettant l'étalonnage et le contrôle de qualité du dosage.

4. Trousse selon l'une quelconque des revendications 1 à 3 comprenant en outre un réactif qui améliore la perméabilité de la membrane cellulaire.

5. Trousse selon l'une quelconque des revendications 1 à 4, dans laquelle le composant (a) est un support solide constitué par une plaque de microtitration dans les puits de laquelle sont fixés le ou les anticorps destinés à immobiliser les cellules parmi lesquelles se trouvent celles de la population ou sous-population contenant l'enzyme à doser.

6. Trousse selon l'une quelconque des revendications 1 à 4, dans laquelle le composé (a) est un support magnétique particulaire.

7. Trousse selon l'une quelconque des revendications 1 à 4, dans laquelle le composant (a) est un tube.

8. Trousse selon l'une quelconque des revendications 1 à 7 dans laquelle le composant (a) est constitué d'un support solide sur lequel sont fixés un ou plusieurs anticorps monoclonaux anti-HLA de classe I.

9. Trousse selon l'une quelconque des revendications 2 à 8, dans laquelle le composant (b) comprend essentiellement de l'eau oxygénée et un chromogène choisi parmi l'orthophénylènediamine, l'acide azino-2,2′ bis (éthyl-3 benzothiazoline sulfonique-6), le diamino-3,3′ benzidine, l'amino-3 éthyl-9 carbazole ou l'un de leurs sels solubles dans l'eau.

10. Procédé de dosage des enzymes endogènes d'une population ou sous-population cellulaire, caractérisé en ce qu'il consiste :
a) à immobiliser la population cellulaire contenant l'enzyme à doser ou une population cellulaire comprenant la sous-population cellulaire contenant l'enzyme endogène à doser sur un support solide en utilisant un ou plusieurs anticorps monoclonaux préalablement fixés par liaison covalente ou par adsorption physique sur ledit support et capable de reconnaître un antigène présent à la surface des cellules ;
b) à observer une période d'incubation ;
c) à laver le support pour éliminer les cellules non immobilisées ;
d) à ajouter une solution du substrat de l'enzyme, tel que défini dans la revendication 1 pour révéler l'activité de l'enzyme endogène ;
e) à lire les résultats par mesure des signaux lumineux (coloration ou fluorescence).

11. Procédé selon la revendication 10, caractérisé en ce qu'à l'étape d), on ajoute en outre un chromogène.

12. Procédé selon la revendication 10 ou la revendication 11, caractérisé en ce que les résultats sont obtenus par référence à une gamme d'étalonnage.

13. Procédé de dosage selon l'une quelconque des revendications 10 à 12, dans lequel le support solide est tel que défini dans l'une des revendications 5, 6 ou 7.

14. Procédé de dosage selon l'une quelconque des revendications 11 à 13, dans lequel l'activité de l'enzyme est révélée par l'eau oxygénée et un chromogène choisi parmi l'orthophénylène diamine, l'acide azino-2,2′ bis (éthyl-3 benzothiazoline sulfonique-6), la diamino-3,3′ benzidine, l'amino-3 éthyl-9 carbazole ou l'un de leurs sels solubles dans l'eau.

15. Procédé selon l'une quelconque des revendications 10 à 14, dans lequel la durée totale d'exécution du dosage est inférieure ou égale à 30 minutes.

16. Procédé selon la revendication 10, dans lequel le ou les anticorps monoclonaux fixés sur le support solide sont des anticorps anti-HLA de classe I.

17. Procédé selon la revendication 11, pour le dosage de la myéloperoxydase endogène des granulocytes du sang humain ou de l'urine.

**Patentansprüche**

1. Kit zur quantitativen Bestimmung von endogenen Enzymen, die für eine Zellpopulation oder eine Zellunterpopulation charakteristisch sind, der folgende Bestandteile enthält:
a) einen festen Träger, an den über kovalente Bindungen oder durch physikalische Adsorption ein oder mehrere monoklonale Antikörper gebunden sind, die gegen Oberflächenantigene der quantitativ zu bestimmenden Zellpopulation gerichtet sind;
b) eine Lösung, die das Substrat enthält, mit dem die Enzymaktivität meßbar ist, wobei dieses Substrat ein Molekül von niederem Molekulargewicht ist, das in das Innere der oben genannten Zellpopulation eindringen kann,

c) eine Pufferlösung zum Waschen.

2. Kit nach Anspruch 1, der zusätzlich ein chromogenes Reagens, mit dem die Enzymaktivität meßbar ist, enthält.

3. Kit nach einem der Ansprüche 1 und 2, der zusätzlich Proben enthält, die eine Eichung und Kontrolle der Qualität der quantitativen Bestimmung erlauben.

4. Kit nach einem der Ansprüche 1 bis 3, der außerdem ein Reagens enthält, durch das die Permeabilität der Zellmembran verbessert wird.

5. Kit nach einem der Ansprüche 1 bis 4, wobei der Bestandteil (a) ein fester Träger ist, der aus einer Mikrotiter-Platte besteht, in deren Vertiefungen der oder die Antikörper gebunden sind, die für die Immobilisierung der Zellen bestimmt sind, unter denen sich Zellen der Population oder der Unterpopulation befinden, die das quantitativ zu bestimmende Enzym enthalten.

6. Kit nach einem der Ansprüche 1 bis 4, wobei der Bestandteil (a) ein teilchenförmiger magnetischer Träger ist.

7. Kit nach einem der Ansprüche 1 bis 4, wobei der Bestandteil (a) ein Rohr ist.

8. Kit nach einem der Ansprüche 1 bis 7, wobei der Bestandteil (a) aus einem festen Träger besteht, an den ein oder mehrere monoklonale Anti-HLA-Antikörper der Klasse I gebunden sind.

9. Kit nach einem der Ansprüche 2 bis 8, wobei der Bestandteil (b) im wesentlichen sauerstoffhaltiges Wasser und ein Chromogen enthält, das unter o-Phenylendiamin, 2,2'-Azino-bis(3-ethylbenzthiazol-6-sulfonsäure), 3,3'-Diaminobenzidin, 3-Amino-9-ethylcarbazol und den wasserlöslichen Salzen dieser Verbindungen ausgewählt ist.

10. Verfahren zur quantitativen Bestimmung von endogenen Enzymen einer Zellpopulation oder einer Zellunterpopulation,
    **gekennzeichnet** durch folgende Schritte:
    a) Immobilisierung der Zellpopulation, die das quantitativ zu bestimmende Enzym enthält, oder einer Zellpopulation, die die Zellunterpopulation einschließt, die das quantitativ zu bestimmende endogene Enzym enthält, auf einem festen Träger unter Verwendung von einem oder mehreren monoklonalen Antikörpern, die zuvor über kovalente Bindungen oder durch physikalische Adsorption an den oben genannten Träger gebunden werden und die ein Antigen zu erkennen vermögen, das an der Oberfläche der Zellen vorhanden ist,
    b) Inkubation während einer bestimmten Dauer,
    c) Waschen des Trägers zur Entfernung der nicht immobilisierten Zellen,
    d) Zugabe einer Lösung des wie in Anspruch 1 definierten Substrats, mit dem die Aktivität des endogenen Enzyms meßbar ist,
    e) Bestimmung der Ergebnisse durch Messen der Lichtsignale (Färbung oder Fluoreszenz).

11. Verfahren nach Anspruch 10, dadurch gekennzeichnet, daß in Schritt d) zusätzlich ein Chromogen zugegeben wird.

12. Verfahren nach Anspruch 10 oder 11, dadurch gekennzeichnet, daß die Ergebnisse gemäß einer Eichkurve erhalten werden.

13. Bestimmungsverfahren nach einem der Ansprüche 10 bis 12, wobei der feste Träger so wie in einem der Ansprüche 5, 6 und 7 definiert ist.

14. Bestimmungsverfahren nach einem der Ansprüche 11 bis 13, wobei die Enzymaktivität mit sauerstoffhaltigem Wasser und einem Chromogen meßbar gemacht wird, das unter o-Phenylendiamin, 2,2'-Azino-bis-(3-ethylbenzthiazol-6-sulfonsäure), 3,3'-Diaminobenzidin, 3-Amino-9-ethylcarbazol und den wasserlöslichen Salzen dieser Verbindungen ausgewählt ist.

15. Verfahren nach einem der Ansprüche 10 bis 14, wobei die Gesamtdauer zur Durchführung der quantitativen Bestimmung kleiner als oder gleich 30 min ist.

16. Verfahren nach Anspruch 10, wobei der oder die an den festen Träger gebundenen monoklonalen Antikörper Anti-HLA-Antikörper der Klasse I sind.

17. Verfahren nach Anspruch 11 zur quantitativen Bestimmung der endogenen Myeloperoxidase von Granulocyten im menschlichen Blut oder im Urin.

## Claims

1. Kit for assaying an endogenous enzyme characteristic of a cell population or subpopulation comprising the following components:
   a) a solid support to which one or more monoclonal antibodies are fixed by covalent bonding or by physical adsorption, said monoclonal antibodies being directed against surface antigens of the cell population to be assayed;
   b) a solution containing the substrate for the enzyme for developing the activity of the enzyme, said substrate being a low-molecular weight molecule capable of penetrating inside said cell population;
   c) a buffer washing solution.

2. Kit according to claim 1 also comprising a chromogenous reagent for developing the activity of the enzyme.

3. Kit according to one of claims 1 and 2 also comprising samples permitting standardization and quality control of the assay.

4. Kit according to any one of claims 1 to 3 also comprising a reagent which improves the permeability of the cell membrane.

5. Kit according to any one of claims 1 to 4 in which component (a) is a solid support consisting of a microtiter plate in whose wells the antibody or antibodies intended for immobilization of the cells, including those of the population or subpopulation containing the enzyme to be assayed, are fixed.

6. Kit according to any one of claims 1 to 4 in which component (a) is a particulate magnetic support.

7. Kit according to any one of claims 1 to 4 in which component (a) is a tube.

8. Kit according to any one of claims 1 to 7 in which component (a) consists of a solid support to which one or more anti-class I HLA monoclonal antibodies are fixed.

9. Kit according to any one of claims 2 to 8 in which component (b) comprises essentially hydrogen peroxide and a chromogen selected from orthophenylenediamine, 2,2′-azino-bis(3-ethylbenzothiazoline-6-sulfonic acid), 3,3′-diaminobenzidine, 3-amino-9-ethylcarbazole or one of their water-soluble salts.

10. Method of assaying the endogenous enzymes of a cell population or subpopulation, characterized in that it consists in:
    a) immobilizing the cell population containing the enzyme to be assayed, or a cell population including the subpopulation containing the endogenous enzyme to be assayed, on a solid support using one or more monoclonal antibodies fixed to said support beforehand by covalent bonding or by physical adsorption and capable of recognizing an antigen present on the surface of the cells;
    b) observing an incubation period;
    c) washing the support to remove the non-immobilized cells;
    d) adding a solution containing the substrate for the enzyme as defined in claim 1 for developing the activity of the enzyme;
    e) reading the results by measuring the light signals (coloration or fluorescence).

11. Method according to claim 10, characterized in that a chromogen is added in step d).

12. Method according to claim 10 or claim 11, characterized in that the results are obtained with reference to a standard scale.

13. Assay method according to any one of claims 10 to 12 in which the solid support is as defined in any one of claims 5, 6 or 7.

14. Assay method according to any one of claims 11 to 13 in which the activity of the enzyme is developed by hydrogen peroxide and a chromogen selected from orthophenylenediamine, 2,2'-azino-bis(3-ethylbenzo-thiazoline-6-sulfonic acid), 3,3'-diaminobenzidine, 3-amino-9-ethyl-carbazole or one of their water-soluble salts.

15. Method according to any one of claims 10 to 14 in which the total time required to perform the assay is less than or equal to 30 minutes.

16. Method according to claim 10 in which the monoclonal antibody or antibodies fixed to the solid support are anti-class I HLA antibodies.

17. Method according to claim 11 for assaying the endogenous myeloperoxidase in the granulocytes of human blood or of urine.